(19) **Europäisches Patentamt**
**European Patent Office**
**Office européen des brevets**

(11) **EP 2 477 691 B1**

(12) **EUROPÄISCHE PATENTSCHRIFT**

(45) Veröffentlichungstag und Bekanntmachung des Hinweises auf die Patenterteilung:
**21.05.2014 Patentblatt 2014/21**

(21) Anmeldenummer: **10766331.2**

(22) Anmeldetag: **13.09.2010**

(51) Int Cl.:
*A61N 1/36* (2006.01)          *A61H 39/00* (2006.01)
*A61N 1/34* (2006.01)

(86) Internationale Anmeldenummer:
**PCT/IB2010/002261**

(87) Internationale Veröffentlichungsnummer:
**WO 2011/030210 (17.03.2011 Gazette 2011/11)**

(54) **GERÄT ZUR PUNKTUAL-STIMULATION**

PUNCTUAL STIMULATION DEVICE

APPAREIL DE STIMULATION PONCTUELLE

(84) Benannte Vertragsstaaten:
**AL AT BE BG CH CY CZ DE DK EE ES FI FR GB GR HR HU IE IS IT LI LT LU LV MC MK MT NL NO PL PT RO SE SI SK SM TR**

(30) Priorität: **14.09.2009 AT 57209 U**

(43) Veröffentlichungstag der Anmeldung:
**25.07.2012 Patentblatt 2012/30**

(73) Patentinhaber: **Szeles, Jozsef Constantin
1190 Wien (AT)**

(72) Erfinder: **Szeles, Jozsef Constantin
1190 Wien (AT)**

(74) Vertreter: **Sonn & Partner Patentanwälte
Riemergasse 14
1010 Wien (AT)**

(56) Entgegenhaltungen:
**WO-A1-86/02567          WO-A2-2004/000413
WO-A2-2008/128270**

• **SATOR-KATZENSCHLAGER S M ET AL: "The Short- and Long-Term Benefit in Chronic Low Back Pain Through Adjuvant Electrical Versus Manual Auricular Acupuncture", ANESTHESIA AND ANALGESIA, WILLIAMS AND WILKINS, BALTIMORE, MD, US, Bd. 98, 1. Januar 2004 (2004-01-01), Seiten 1359-1364, XP002488596, ISSN: 0003-2999, DOI: DOI:10.1213/01.ANE. 0000107941.16173.F7**
• **KANIUSAS E ET AL: "Adaptive auricular electrical stimulation controlled by vital biosignals. Transition from fixed to adaptive and synchronized electrical stimulation controlled by heart rate variability and blood perfusion", PROCEEDINGS OF THE SECOND INTERNATIONAL CONFERENCE ON BIOMEDICAL ELECTRONICS AND DEVICES, BIODEVICES 2009; PORTO, PORTUGAL, JANUARY 14-17, 2009, INSTICC PR, PT, 14. Januar 2009 (2009-01-14), Seiten 304-309, XP009141309, ISBN: 978-989-8111-64-7**

**Beschreibung**

Die Erfindung bezieht sich auf ein Gerät gemäß dem Oberbegriff des Anspruchs 1.

Ein ähnliches Gerät, bei dem zur Stimulation von im Bereich der Ohren liegenden Nervenendigungen Flächenelektroden vorgesehen sind und alternativ auch eine Stimulation über einen transcranial den Schädel durchquerenden Stromweg vorgesehen ist, ist in der WO 86/02567 A1 beschrieben. Weiters geht ein ähnliches Gerät, bei dem ein einziger, den Behandlungsstrom führender Ausgangskanal vorgesehen ist, an den mehrere flexible Leitungen angeschlossen sind, die zur Verbindung mit je einer Stimulations-Nadelelektrode dienen, aus der Publikation: "The Short-and Long-Term Benefit in Chronic Low Back Pain Through Adjuvant Electrical Versus Manual Auricular Acupuncture" von Sator-Katzenschlager S M et al., veröffentlicht in "Anesthesia and Analgesia" Vol 98 pages 1359-1364 hervor.

Es ist ein Ziel der Erfindung ein Gerät vorstehend angeführter Art zu schaffen, welches hinsichtlich der Details der Stimulation ein größeres Spektrum an Möglichkeiten bietet und damit einen breiteren Bereich der therapeutischen Anwendung erzielen lässt. Auch soll bei dem zu schaffenden Gerät die Wirkung von Einflüssen, die die Stimulation beeinträchtigen können, z.B. die Wirkung von Kräften, die von Außen auf die Elektroden einwirken, oder die Wirkung von Veränderungen der im Elektrodenbereich vorliegenden Widerstandswerte, klein gehalten oder ausgeglichen werden können.

Das erfindungsgemäße Gerät eingangs erwähnter Art ist dadurch gekennzeichnet, dass in der Mikrocomputerschaltung ein durch externe Betätigung wahlweise aktivierbarer Adaptivstrom-Steuerkreis vorgesehen ist, der mit einem internen Speicher für Behandlungsstromparameter wirkverbunden ist und über einen Digital-Analog-Wandler, der an einen Digitalanschluss der Mikrocomputerschaltung angeschlossen ist, mindestens einem in einem Ausgangskanal vorgesehenen, für bipolare Ausgangssignale ausgebildeten Verstärker ein Steuersignal zuführt, das in dem mit einer Stimulationselektrode verbundenen Ausgangskreis dieses Verstärkers ein dem in diesem Ausgangskreis jeweils vorliegenden Widerstandswert adaptiv folgendes Verhalten von Stromstärke und Stimulationsspannung entsprechend den Beziehungen

$$I = k \cdot R$$

$$U = k \cdot R^2$$

herbeiführt, wobei k eine wählbare Konstante ist. Durch diese Ausbildung kann der vorstehend angeführten Zielsetzung gut entsprochen werden. Es kann z.B. bei einer von Außen verursachten Lockerung eines zunächst elektrisch gut leitenden Sitzes einer Stimulationselektrode oder z.B. bei einem starken Anstieg des elektrischen Widerstandes des Unterhautgewebes an der Einstichstelle einer Stimulationselektrode, die stimulierende Funktion der betreffenden Stimulationselektrode beeinträchtigt sein, aber weiterhin eine, wenn auch eingeschränkte, Stimulation mit einer anderen Stimulationselektrode, die ja an einen eigenen Ausgangskanal angeschlossen ist, erfolgen. Auch bei funktionellen Fehlern im Bereich einer Bezugselektrode kann eine Stimulation durch entsprechende Steuerung der voneinander unabhängigen Ausgangskanäle aufrecht erhalten werden, indem ein Stromfluss der über zwei oder mehr Stimulationselektroden führt, hergestellt wird. Das Vorhandensein mehrerer Ausgangskanäle gibt auch die Möglichkeit, an verschiedenen Rezeptorenbereichen, an denen sich unterschiedliche Nervenendigungen befinden, unabhängig voneinander mit Behandlungsströmen, die sich in ihren Parametern unterscheiden, zu stimulieren. So kann man an verschiedenen Stellen des Ohres eine Stimulation mit unterschiedlicher Intensität vorsehen und man kann z.B. parasympathische und sympathische Nervenstränge selektiv stimulieren. Es können die Parameter der Behandlungsströme auf einfache Weise in breiten Bereichen gewählt bzw. eingestellt werden, wobei z.B. ein Wechsel erfolgen kann von Behandlungsströmen hoher Intensität, die aus einer Reihe von Impulspaketen gebildet sind und sich besonders zur Schmerztherapie im Akutstadium eignen, zu Behandlungsströmen niedriger Intensität, die aus Einzelimpulsen gebildet sind und sich besonders zur Schmerztherapie bei chronischen Schmerzen eignen, wobei auch im Verlauf einer solchen Behandlung an deren Beginn ein langsamer Anstieg der Intensität und an deren Ende ein langsames Abklingen vorgesehen werden kann.

Die Erfassung der im Zuge einer Stimulationsbehandlung jeweils auftretenden Werte der den Stimulationselektroden zugeführten Ströme und der an diesen Stimulationselektroden jeweils vorliegenden Spannungen ist für die Programmierung der Parameter der Behandlungsströme und für deren Aufrechterhaltung im Zuge der Stimulation von Vorteil. Hierzu ist gemäß bevorzugter Ausführungsformen des erfindungsgemäßen Gerätes einerseits vorgesehen, dass in den Ausgangskanälen in die zu den Stimulationselektroden führenden Stromwege Messwiderstände zur Strommessung eingefügt sind und an die einzelnen Messwiderstände jeweils ein Messverstärker angeschlossen ist, dessen Ausgangssignal ein Maß für die Stromstärke des Behandlungsstromes ist und der Mikrocomputerschaltung zugeleitet wird, und andererseits vorgesehen, dass an mindestens einem Ausgangskanal eine Spannungsmessschaltung vorgesehen ist, welche an die Mikrocomputerschaltung ein Messsignal abgibt, das der Spannung entspricht, die zwischen der dem betreffenden Ausgangskanal zugeordneten Stimulationselektrode und einem im Behandlungsstromgenerator befindlichen Bezugspotential-Punkt, der mit einer Bezugselektrode verbunden ist, vorliegt.

Dabei sieht man vorteilhaft vor, dass in den Adaptivstrom-Steuerkreis ein Adaptivstrom-Regelkreis eingebunden ist, dem ein aus dem Messsignal der Spannungsmessschaltung und dem Ausgangssignal des Messverstärkers der Strommessung gewonnener Widerstandsmesswert als Istsignal zugeführt wird.

Insbesondere für ein Gleichbleiben der die Stimulation begleitenden Empfindungen ist dabei eine Ausbildung des vorgenannten erfindungsgemäßen Gerätes vorteilhaft, welche dadurch gekennzeichnet ist, dass der in der Mikrocomputerschaltung vorgesehene Steuerkreis den in den Ausgangskanälen vorgesehenen Verstärkern ein Steuersignal zuführt, das in zyklischer Aufeinanderfolge Zeitintervalle, in denen ein Stromfluss zu den Stimulationselektroden erfolgt, und Zeitintervalle, in denen die Ausgangskanäle inaktiv sind, bildet, wobei jeweils ein Stromflusszeitintervall und ein Inaktiv-Zeitintervall zusammen einen Stimulationszyklus bilden, und das eine Variation des über die Zeitdauer eines Stimulationszyklus bestimmten Mittelwertes der Stromstärke durch Variation dieser Zeitdauer durch das Steuersignal herbeiführt.

Eine Ausbildung des erfindungsgemäßen Gerätes, welche insbesondere hinsichtlich einer wählbaren Einstellung der Intensität der Behandlungsströme vorteilhaft ist und welche auch den Vorteil bietet, dass bei einem sich unbeabsichtigt ergebenden Anstieg des elektrischen Widerstandes in den über die Stimulationselektroden führenden Stromkreisen, welcher Anstieg sich durch Widerstandserhöhungen in der Haut oder im Unterhautgewebe oder bei einer Lockerung des Sitzes einer Stimulationselektrode ergeben kann, keine Spannungsspitzen entstehen, welche unter Umständen unangenehm empfunden werden, ist dadurch gekennzeichnet, dass in der Mikrocomputerschaltung ein durch externe Betätigung wahlweise aktivierbarer Konstantspannungs-Steuerkreis vorgesehen ist, der mit einem internen Speicher für Behandlungsstromparameter wirkverbunden ist und über einen Digital-Analog-Wandler mindestens einem in einem Ausgangskanal vorgesehenen Verstärker ein Steuersignal zuführt, das in dem mit einer Stimulationselektrode verbundenen Ausgangskreis dieses Verstärkers das Verhalten einer hinsichtlich des Spannungswertes einstellbaren Konstantspannungsquelle herbeiführt. Hierbei ist es weiter vorteilhaft, wenn man vorsieht, dass in den Konstantspannungs-Steuerkreis ein Konstantspannungs-Regelkreis eingebunden ist, dem das Messsignal der betreffenden Spannungsmessschaltung als Istsignal zugeführt wird.

Steht die Aufrechterhaltung einer bestimmten gewählten Intensität des Behandlungsstromes im Vordergrund, ist eine Ausführungsform des erfindungsgemäßen Gerätes vorteilhaft, welche dadurch gekennzeichnet ist, dass in der Mikrocomputerschaltung ein durch externe Betätigung wahlweise aktivierbarer Konstantstrom-Steuerkreis vorgesehen ist, der mit einem internen Speicher für Behandlungsstromparameter wirkverbunden ist und über einen Digital-Analog-Wandler mindestens einem in einem Ausgangskanal vorgesehenen Verstärker ein Steuersignal zuführt, das in dem mit einer Stimulationselektrode verbundenen Ausgangskreis dieses Verstärkers das Verhalten einer hinsichtlich der Stromstärke einstellbaren Konstantstromquelle herbeiführt, und in den Konstantstrom-Steuerkreis ein Konstantstrom-Regelkreis eingebunden ist, dem das Ausgangssignal des Messverstärkers als Istsignal zugeführt wird.

Es ist für eine Stimulationsbehandlung bei bestimmten Gesundheitsstörungen bzw. Krankheiten und z.B. zum Erzielen einer homogenen Empfindung am ganzen Ohr vorteilhaft, die Polarität der Behandlungsströme fortlaufend zu wechseln. Hierzu ist eine Ausbildung des erfindungsgemäßen Gerätes vorgesehen, bei der während eines Stromflusses der den einzelnen Stimulationselektroden zugeführten Behandlungsströme diese Stimulationselektroden im jeweils betrachteten Zeitpunkt bezüglich einem mit den Behandlungsstromkreisen in Verbindung stehenden Bezugspotential-Punkt voneinander unterschiedliche Polarität aufweisen. Diese Ausbildung ergibt auch eine mindestens teilweise Entlastung einer mit dem Bezugspotential-Punkt verbundenen Bezugselektrode von den über die Stimulationselektroden fließenden Behandlungsströmen. Diese Wirkung kommt in noch stärkerem Maße bei einer Weiterbildung zum Tragen, bei der die den einzelnen Stimulationselektroden, die im jeweils betrachteten Zeitpunkt voneinander unterschiedliche Polarität aufweisen, zugeführten Behandlungsströme im jeweils betrachteten Zeitpunkt einander kompensieren und so kein Behandlungsstrom über eine Bezugselektrode fließt. Es kann so eine Bezugselektrode erübrigt werden. Es ist weiter günstig wenn man vorsieht, dass der in der Mikrocomputerschaltung vorgesehene Steuerkreis den in den Ausgangskanälen vorgesehenen Verstärkern ein Steuersignal zuführt, das in zyklischer Aufeinanderfolge Zeitintervalle, in denen ein Stromfluss zu den Stimulationselektroden erfolgt, und Zeitintervalle, in denen die Ausgangskanäle inaktiv sind, bildet, und im Verlauf jedes Stromflusszeitintervalls einen Polaritätswechsel herbeiführt, wobei jeweils ein Stromflusszeitintervall und ein Inaktiv-Zeitintervall zusammen einen Stimulationszyklus bilden und in den Stromflusszeitintervallen ein Behandlungsstrom in Form eines Paketes aufeinander folgender Impulse oder in Form eines Einzelimpulses fließt. Vorzugsweise ist dabei vorgesehen, dass der Polaritätswechsel im Verlauf der Stromflusszeitintervalle herbeigeführt wird. Dies wird therapeutisch oft als günstig angesehen.

Aus therapeutischer Sicht und auch hinsichtlich einer Minderung oder auch Vermeidung eines Behandlungsstromflusses über eine Bezugselektrode ist eine Ausbildung des erfindungsgemäßen Gerätes vorteilhaft, welche dadurch gekennzeichnet ist, dass der in der Mikrocomputerschaltung vorgesehene Steuerkreis, der den in den Ausgangskanälen vorgesehenen Verstärkern ein Steuersignal zuführt, das in zyklischer Aufeinanderfolge Zeitintervalle, in denen ein Stromfluss zu den Stimulationselektroden erfolgt, und Zeitintervalle, in denen die Ausgangskanäle inaktiv sind, bildet, wobei jeweils ein Stromflusszeitintervall und ein Inaktiv-Zeitintervall zusammen einen Stimulationszyklus bilden, im Zuge der einzelnen Stromflusszeitintervalle eine oder mehrere Veränderungen der Intensität der den einzelnen Stimulationselektroden zugeführten Behandlungsströme herbeiführt, wobei die an einer jeweils betrachteten Stimulationselektrode erfolgenden Intensitätsveränderungen gegenläufig zu den an einer oder mehreren anderen jeweils betrachteten Stimulationselektrode erfolgenden Intensitätsveränderungen verlaufen. Es kann dies schon mit zwei Ausgangskanälen und Stimulationselektroden realisiert werden, es erscheint aber besser mit drei oder mehr Ausgangskanälen und Stimulationselektroden gangbar. Eine bevorzugte Ausbildung ist dabei dadurch gekennzeichnet, dass drei je einer eigenen Stimulationselektrode zugeordnete Ausgangskanäle vorgesehen sind und in den Stromflusszeitintervallen in jedem jeweils betrachteten Zeitpunkt der jeder einzelnen Stimulationselektrode zugeführte Behandlungsstrom hinsichtlich Intensität und Polarität durch die den jeweils beiden anderen Stimulationselektroden zugeführten Behandlungsströme kompensiert ist.

Besonders auf eine Schmerztherapie in chronischen Fällen abgestimmt, aber auch in anderen Fällen günstig, ist eine Ausbildung des Gerätes, welche dadurch gekennzeichnet ist, dass die den einzelnen Stimulationselektroden zugeführten Behandlungsströme in jedem Stromflusszeitintervall durch einen Einzelimpuls gebildet sind und in jedem dieser Einzelimpulse zeitlich aufeinander folgend mehrere Intensitätsveränderungen und mindestens ein Polaritätswechsel vorgesehen sind. Hierbei ist eine Realisierung im Zusammenhang mit drei Ausgangskanälen bevorzugt. Für das Erzielen der erwähnten gegenseitigen Kompensation der Behandlungsströme ist es günstig, wenn man vorsieht, dass die in den einzelnen Stromflusszeitintervallen vorgesehene ein- oder mehrmalige Veränderung der Behandlungsstromintensität stufenweise erfolgt.

Wie bereits oben erwähnt, ist es bei bestimmten Stimulations therapien, z.B. bei Schmerztherapien für chronische Beschwerden, vorteilhaft, am Beginn einer Behandlung die Intensität der Stimulation von kleinen Werten ausgehend langsam auf das volle vorgesehene Ausmaß zu erhöhen. Hierfür ist eine Ausführungsform des erfindungsgemäßen Gerätes vorgesehen, die dadurch gekennzeichnet ist, dass der in der Mikrocomputerschaltung vorgesehene Steuerkreis den in den Ausgangskanälen vorgesehenen Verstärkern ein Steuersignal zuführt, das in zyklischer Aufeinanderfolge Zeitintervalle, in denen ein Stromfluss zu den Stimulationselektroden erfolgt, und Zeitintervalle, in denen die Ausgangskanäle inaktiv sind, bildet, wobei jeweils ein Stromflusszeitintervall und ein Inaktiv-Zeitintervall zusammen einen Stimulationszyklus bilden, und am Beginn einer durch eine Vielzahl von aufeinander folgenden Stimulationszyklen gebildeten Stimulationszyklenfolge während mehrerer aufeinander folgender Stimulationszyklen die Intensität des einer jeweils betrachteten Stimulationselektrode zugeführten Behandlungsstromes, als Mittelwert über das jeweilige Stromflusszeitintervall betrachtet, fortlaufend von jeweils einem Stimulationszyklus zum darauf folgenden Stimulationszyklus erhöht und danach während einer Vielzahl aufeinander folgender Stimulationszyklen in deren Stromflusszeitintervallen den zuvor erreichten Pegel der Intensität der Behandlungsströme aufrecht erhält. Es kann dabei sowohl das Empfinden der Stimulation als auch die Wirkung der Behandlung weiter verbessert werden, wenn man vorsieht, dass am Ende der Stimulationszyklenfolge der Steuerkreis den in den Ausgangskanälen vorgesehenen Verstärkern ein Steuersignal zuführt, das die Intensität des einer jeweils betrachteten Stimulationselektrode zugeführten Behandlungsstromes, als Mittelwert über das jeweilige Stromflusszeitintervall betrachtet, fortlaufend von jeweils einem Stimulationszyklus zum darauf folgenden Stimulationszyklus vermindert.

Hinsichtlich der Energieversorgung des erfindungsgemäßen Gerätes ist eine Ausführungsform bevorzugt, welche dadurch gekennzeichnet ist, dass zur Speisung der in den Ausgangskanälen vorgesehenen Verstärker ein Spannungswandler mit bipolarem Ausgang vorgesehen ist, der seinerseits von der im Gerät vorgesehenen Batterie gespeist wird. Es kann so die Speisung der Verstärker mit einer Batterie erfolgen, die nur aus einer Zelle oder wenigen Zellen gebildet ist und bei gegebenen Raum einen verhältnismäßig großen Energieinhalt besitzt, der einen Stimulationsbetrieb über eine Reihe von Tagen ermöglicht. Eine weitere Verlängerung der mit dem Gerät möglichen Behandlungsdauer kann durch Minimierung der im genannten Spannungswandler entstehenden Verluste erzielt werden. Hierzu sieht eine Weiterbildung der vorgenannten Ausbildungsform vor, dass in die von der Batterie zum Spannungswandler führende speisende Verbindung eine Schalteinrichtung eingefügt ist, welche die Speisung des Spannungswandlers sowohl während in den Behandlungsstromkreisen vorliegender Stromflusspausen als auch bei Überschreitung eines vorgegebenen Grenzwertes des der Batterie vom Spannungswandler entnommenen Stromes unterbricht. Die Schalteinrichtung hat dabei neben der energiesparenden Funktion die Rolle einer Sicherung gegen Überlastung der Batterie und gegen eine Überschreitung vorgegebener Werte der Behandlungsströme.

Eine weitere Ausführungsform, die im Sinne einer möglichst guten Ausnützung der in der speisenden Batterie enthaltenen Energie wirksam ist, ist dadurch gekennzeichnet, dass zur Speisung der Mikrocomputerschaltung, des Digital-Analog-Wandlers und von im Gerät vorgesehener Messsignalschaltungen ein an die Batterie angeschlossener Spannungswandler mit unipolarem Ausgang vorgesehen ist, der mit einer Schottky-Diode überbrückt ist und nur bei Absinken der Batteriespannung aktiviert wird und an seinem Ausgang eine der Batterie-Sollspannung entsprechende Spannung abgibt.

Für die Einstellung der Parameter der Behandlungsströme an den jeweils vorliegenden Behandlungsfall ist eine Ausführungsform des erfindungsgemäßen Gerätes vorteilhaft und bevorzugt, welche dadurch gekennzeichnet ist, dass das Gerät eine an die Mikrocomputerschaltung angeschlossene drahtlos arbeitende Übertragungseinrichtung zur Eingabe von zu speichernden Parametern der für die jeweilige Behandlung vorgesehenen Behandlungsströme mittels eines externen Steuergerätes aufweist. Man kann weiter vorteilhaft vorsehen, dass das Gerät eine an die Mikrocomputerschaltung angeschlossene drahtlos arbeitende Übertragungseinrichtung zur Übermittlung von gespeicherten Parametern der für die jeweilige Behandlung vorgesehenen Behandlungsströme an ein externes Steuer- und Kontrollgerät aufweist.

Als externes Steuergerät kann ein üblicher Computer eingesetzt werden, der eine Schnittstelle besitzt, an die ein mit einer Sende- und Empfangseinrichtung versehener Adapter anschließbar ist.

Zur drahtlosen Übertragung können vorteilhaft Einrichtungen eingesetzt werden, die in einem für Steuerungszwecke vorgesehenen Band im Dezimeterwellenbereich arbeiten. Es können aber auch Einrichtungen Verwendung finden, bei denen die Übertragung der Steuerungsinformationen im Infrarotbereich erfolgt. Es sind auch einfachere Ausführungsformen möglich, bei denen Steuerinformationen, welche den Parametern der vorgesehenen Behandlungsströme entsprechen, im Zuge der Herstellung eines solchen Gerätes eingespeichert werden.

Die Erfindung wird nun anhand von Beispielen unter Bezugnahme auf die Zeichnung, in der Ausführungsbeispiele des Erfindungsgegenstandes schematisch dargestellt sind, weiter erläutert. In der Zeichnung zeigt

Fig. 1. ein Ausführungsbeispiel eines erfindungsgemäß ausgebildeten Gerätes in blockschematischer Darstellung.

Die Fig. 2, 3 und 4 zeigen Diagramme, die verschiedene Arten der Steuerung von zur Stimulation vorgesehenen Behandlungsströmen verdeutlichen.

Die Fig. 5 bis 10 zeigen Zeitdiagramme, die den Verlauf von zur Stimulation vorgesehenen Behandlungsströmen darstellen, welche bei verschiedenen Ausführungsformen des erfindungsgemäßen Gerätes auftreten.

Fig. 11 zeigt ein Blockschaltbild einer Ausführungsform einer bei einem erfindungsgemäßen Gerät vorgesehenen Spannungsversorgungsschaltung.

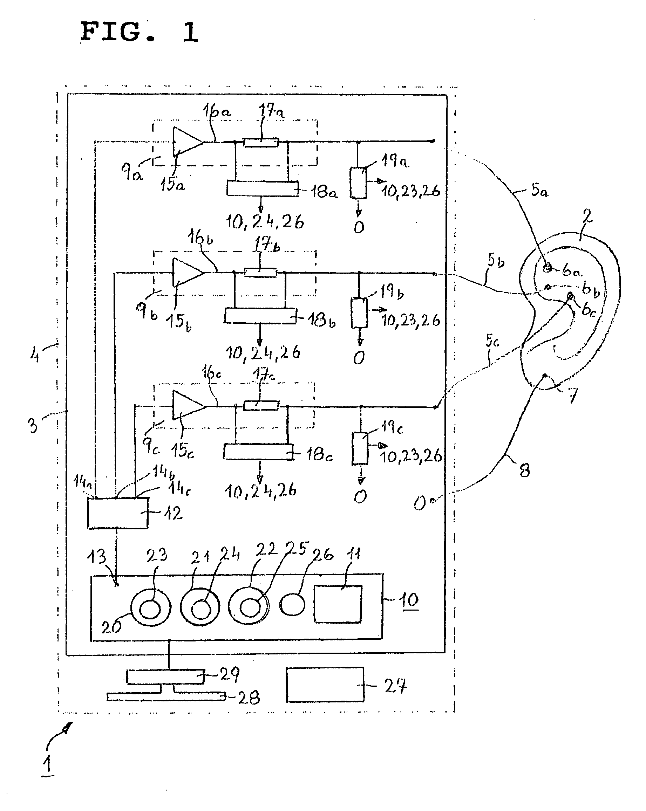

Das in Fig. 1 in blockschematischer Form dargestellte Ausführungsbeispiel eines erfindungsgemäß ausgebildeten Gerätes ist zur Punktual-Stimulation von im Bereich der Ohren liegenden Endigungen von Nerven vorgesehen, die zu Hirnstammkernen führen. Dieses Gerät 1 weist einen batteriegespeisten Behandlungsstromgenerator 3 auf, der in einem Gehäuse 4 angeordnet ist, das am Körper im Bereich eines Ohres 2, an dem die Stimulation vorgenommen werden soll, zu tragen ist. Hinsichtlich der Abmessungen des Gerätes 1 ist zu erwähnen, dass die Darstellung in Fig. 1 primär die baulichen und funktionellen Merkmale verdeutlichen soll und die Abmessungen des Gehäuses 4, das die Bauteile des Gerätes umgibt, in der Regel kleiner als die Abmessungen des Ohres 2 gehalten werden können. Der batteriegespeiste Behandlungsstromgenerator 3 beinhaltet eine niederfrequente behandlungsstromformende Elektronikschaltung, welche über flexible Leitungen 5a, 5b, 5c im Ohr 2 anzuordnende Stimulations-Nadelelektroden 6a, 6b, 6c speist. Eine im dargestellten Fall als Nadelelektrode 7 ausgebildete Bezugselektrode ist über eine flexible Verbindung 8 an einen Bezugspotential-Punkt 0 des Gerätes angeschlossen. Die Bezugselektrode 7 kann auch in anderer Form ausgebildet sein, z.B. in Form einer Oberflächenelektrode, welche, wie dargestellt, an einer flexiblen Verbindung 8 angeordnet sein kann oder auch in mechanisch anderer Weise am Gehäuse 4 des Gerätes sitzen kann. Im Rahmen der erwähnten Elektronikschaltung weist der Behandlungsstromgenerator 3 mehrere analog arbeitende Ausgangskanäle 9a, 9b, 9c auf, wobei jeder dieser Ausgangskanäle einer eigenen Stimulationselektrode 6a, 6b, 6c zugeordnet ist und den Behandlungsstrom an diese Elektroden liefert. Die genannten analog arbeitenden Ausgangskanäle sind eingangsseitig, je für sich an eine Mikrocomputerschaltung 10 angeschlossen, in der Parameterwerte der für die jeweilige Behandlung vorgesehenen Behandlungsströme zur fortlaufenden Auslesung in einem Speicher 11 speicherbar sind. In den einzelnen Ausgangskanälen 9a, 9b, 9c ist je ein, von einem Digital-Analog-Wandler 12 gesteuerter Verstärker 15a, 15b, 15c vorgesehen, der für bipolare Ausgangssignale ausgebildet ist. Baulich vorteilhaft sind die Digital-Analog-Wandler zu einer Einheit zusammengefasst, können aber auch getrennt realisiert sein. Der Digital-Analog-Wandler 12 ist seinerseits an einen Digitalanschluss 13 der Mikrocomputerschaltung 10 angeschlossen. Es sind mindestens zwei der genannten Ausgangskanäle, die je einer eigenen Stimulationselektrode zugeordnet sind, vorgesehen, vorzugsweise, wie dargestellt, drei derartige Ausgangskanäle. Man kann aber auch gewünschtenfalls eine größere Anzahl solcher Ausgangskanäle und damit verbundener Stimulationselektroden vorsehen. Als Verstärker 15a, 15b, 15c sieht man vorzugsweise Operationsverstärker vor.

In den Ausgangskanälen 9a, 9b, 9c sind in die zu den Stimulationselektroden 6a, 6b, 6c führenden Stromwege 16a, 16b, 16c Messwiderstände 17a, 17b, 17c eingefügt, die zur Messung der Intensität der Behandlungsströme dienen. An die einzelnen Messwiderstände 17a, 17b, 17c ist jeweils ein Messverstärker 18a, 18b, 18c angeschlossen, dessen Ausgangssignal ein Maß für die Stromstärke des Behandlungsstromes ist und es wird dieses Ausgangssignal der Mikrocomputerschaltung 10 zugeleitet. An den Ausgangskanälen 9a, 9b, 9c sind auch Spannungsmessschaltungen 19a, 19b, 19c vorgesehen, deren jede an die Mikrocomputerschaltung 10 ein Messsignal abgibt, das der Spannung entspricht, die zwischen der dem betreffenden Ausgangskanal zugeordneten Stimulationselektrode 6a, 6b, 6c und dem Bezugspotential 0 bzw. der Bezugselektrode 7 vorliegt.

In der Mikrocomputerschaltung 10 ist ein durch externe Betätigung wahlweise aktivierbarer Konstantspannungs-Steuerkreis 20 vorgesehen, der mit dem internen Speicher 11 für Behandlungsstromparameter wirkverbunden ist und über den Digital-Analog-Wandler 12 mindestens einem Verstärker 15a, 15b, 15c, die je in einem Ausgangskanal 9a, 9b, 9c vorgesehen sind, über die Ausgänge 14a, 14b, 14c des Digital-Wandlers 12 ein Steuersignal zuführt, das in dem mit einer Stimulationselektrode 6a, 6b, 6c verbundenen Ausgangskreis des jeweiligen Verstärkers 15a, 15b, 15c das Verhalten einer, hinsichtlich des Spannungswertes einstellbaren, Konstantspannungsquelle herbeiführt. Vorzugsweise ist dabei wie in Fig. 1 dargestellt vorgesehen, dass in den Konstantspannungs-Steuerkreis 20 ein Konstantspannungs-Regelkreis 23 eingebunden ist, dem das Messsignal der betreffenden Spannungsmessschaltung 19a, 19b, 19c als Istsignal zugeführt wird. Weiter ist ein durch externe Betätigung wahlweise aktivierbarer Konstantstrom-Steuerkreis 21 vorgesehen, der gleichfalls mit dem internen Speicher 11 für Behandlungsstromparameter wirkverbunden ist und über den Digital-Analog-Wandler 12, an dessen Ausgänge 14a, 14b, 14c die Verstärker 15a, 15b, 15c angeschlossen sind, mindestens einem dieser Verstärker ein Steuersignal zuführt, das in dem mit einer Stimulationselektrode 6a, 6b, 6c verbundenen Ausgangskreis dieses Verstärkers 15a, 15b, 15c das Verhalten einer hinsichtlich der Stromstärke einstellbaren Konstantstromquelle herbeiführt; hierbei ist in den Konstantstrom-Steuerkreis 21 ein Konstantstrom-Regelkreis 24 eingebunden, dem das Ausgangssignal des zum jeweils in Betracht gezogenen Verstärker 15a, 15b, 15c zugeordneten Messverstärkers 18a, 18b, 18c als Istsignal zugeführt wird.

Weiters ist in der Mikrocomputerschaltung 10 ein durch externe Betätigung wahlweise aktivierbarer Adaptivstrom-Steuerkreis 22 vorgesehen, der mit dem internen Speicher 11 für Behandlungsstromparameter wirkverbunden ist und mindestens einem in einem Ausgangskanal 9a, 9b, 9c vorgesehenen Verstärker 15a, 15b, 15c ein Steuersignal zuführt, dass in dem mit einer Stimulationselektrode 6a, 6b, 6c verbundenen Ausgangskreis des betreffenden Verstärkers 15a, 15b, 15c ein dem in diesem Ausgangskreis jeweils vorliegenden Widerstandswert adaptiv folgendes Verhalten von Stromstärke und Stimulationsspannung entsprechend den Beziehungen $I = k \cdot R$ und $U = k \cdot R^2$ herbeiführt, wobei k eine wählbare Konstante ist. Vorzugsweise ist dabei in den Adaptivstrom-Steuerkreis 22 ein Adaptivstrom-Regelkreis 25 eingebunden, dem ein aus dem Messsignal der jeweiligen Spannungsmessschaltung 19a, 19b, 19c und dem Ausgangssignal des jeweiligen Messverstärkers 18a, 18b, 18c der Strommessung gewonnener Widerstandsmesswert als Istsignal zugeführt wird. Zur Bildung des Widerstandsmesswertes ist in der Mikrocomputerschaltung 10 ein Widerstandsrechenkreis 26 vorgesehen.

Die Widerstandsbestimmung oder auch eine Spannungsmessung mit einer oberwähnten Spannungsmessschaltung kann auch zur Kontrolle, ob die Stimulationselektroden beim Platzieren korrekt angeordnet worden sind, angewendet werden.

Im Sinne einer vielfältigen Einsetzbarkeit des Gerätes kann man, wie in Fig. 1 dargestellt, in der Mikrocomputerschaltung 10 die drei verschiedenen Steuerkreise 20, 21, 22 vorsehen; man kann aber auch im Sinne einer Vereinfachung bzw. im Sinne einer Ausrichtung des Gerätes auf bestimmte Therapieformen, auch nur einen oder zwei dieser Steuerkreise vorsehen. Im Gehäuse 4 des Gerätes kann man auch, wie dargestellt, die Speisebatterie 27 und die z.B. mit einer Antenne 28 ausgestattete drahtlos arbeitende Übertragungseinrichtung 29, die für die Eingabe von zu speichernden Parametern der für die jeweilige Behandlung vorgesehenen Behandlungsströme dient und die ihrerseits an die Mikrocomputerschaltung 10 angeschlossen ist, vorsehen.

Es ist baulich und funktionell vorteilhaft, wenn man vorsieht, dass die Mikrocomputerschaltung 10 mit ihrem Speicher 11 und Steuerkreisen 20, 21, 22, 26, und vorzugsweise auch die die Ausgangskanäle 9a, 9b, 9c bildenden und diesen Ausgangskanälen zugeordneten Bauelemente, in einem Mikrochip integriert sind. Auch Bauelemente, die zur drahtlosen Übertragung von Parametern von Behandlungsströmen dienen, können in eine solche Integration eingebunden sein.

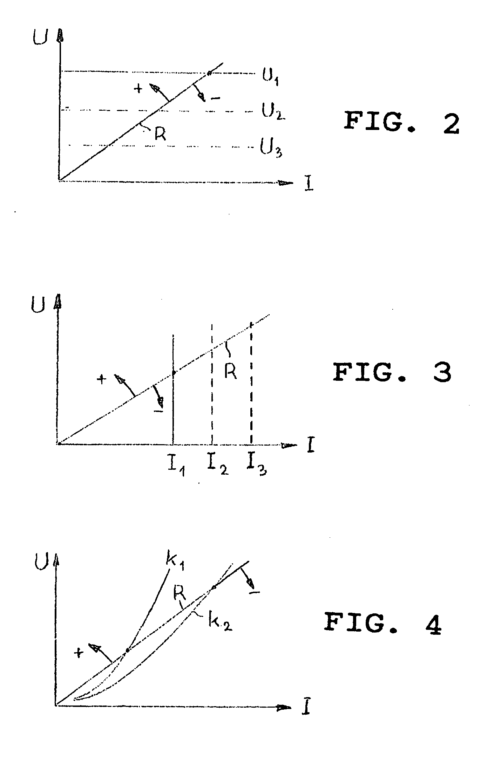

Die Fig. 2, 3 und 4 verdeutlichen den Zusammenhang zwischen dem an den Elektroden wirksamen Werten von Strom, Spannung und dem elektrischen Widerstand, der im jeweiligen Stromkreis, der von dem im jeweiligen Ausgangskanal liegenden Verstärker über die zugeordnete Stimulationselektrode führt, bei verschiedenen Betriebsformen vorliegt.

Fig. 2 zeigt diesen Zusammenhang, wenn der im genannten über eine Stimulationselektrode führenden Stromkreis, der im jeweiligen Ausgangskanal vorliegende Verstärker das Verhalten einer einstellbaren Konstantspannungsquelle aufweist. Es sind mehrere Spannungswerte, welche gewählt werden können, mit U1 U2 und U3 bezeichnet und es ergibt sich der jeweils fließende Strom aus dem Schnittpunkt der mit R bezeichneten Widerstandsgeraden mit dem jeweils eingestellten Spannungswert. An der Stimulationselektrode liegt die jeweils eingestellte Spannung (U1, U2, U3) an und es nimmt der über die Stimulationselektrode fließende Strom I mit steigendem Widerstand im Kreis der Stimulationselektrode ab und mit sinkendem Widerstand zu. Ein Anstieg des Widerstandes R ist in Fig. 2 mit + und eine Verminderung des Widerstandes R mit - angedeutet.

Fig. 3 verdeutlicht die Zusammenhänge, wenn im jeweiligen mit einer Stimulationselektrode verbundenem Ausgangskreis eines in einen Ausgangskanal vorgesehenen Verstärkers das Verhalten einer hinsichtlich der Stromstärke einstellbaren Konstantstromquelle vorliegt. Es sind drei wahlweise einstellbare Stromwerte I1, I2, I3 angedeutet. Die an der jeweils betrachteten Stimulationselektrode auftretende Spannung steigt mit steigendem Widerstand und fällt mit fallendem Widerstand.

Fig. 4 zeigt die Zusammenhänge bei einem dem jeweils im Stromkreis einer Stimulationselektrode vorliegenden Widerstandswert adaptiv folgendem Verhalten von Stromstärke und Stimulationsspannung bei einer adaptiven Steuerung des Verstärkers entsprechend den Beziehungen $I = k \cdot R$ und $U = k \cdot R^2$. Die jeweils zusammengehörenden Werte der Stromstärke des über die betreffende Stimulationselektrode fließenden Stromes und der an der Stimulationselektrode wirksamen Spannung liegen auf parabelartigen Kennlinien und es sind in Fig. 4 zwei derartige Kennlinien, welche für voneinander verschiedene Werte von k gelten, eingezeichnet und mit k1 und k2 bezeichnet. Die sich bei einem bestimmten Wert des Widerstandes in dem über die betreffende Stimulationselektrode führenden Stromkreis ergebenden Werte der Stromstärke und der Stimulationsspannung sind durch den jeweils vorliegenden Schnittpunkt der Widerstandsgeraden R mit der dem jeweiligen Wert k entsprechenden Kennlinie gegeben. Bei einer Verminderung des Widerstandswertes nehmen Strom und Spannung im Stimulationsstromkreis ab und umgekehrt erhöhen sich Strom und Spannung im Stimulationsstromkreis bei einer Zunahme des im Stimulationsstromkreis wirksamen Widerstandes. Ein solches adaptives Verhalten der Werte von Strom und Spannung an der jeweils betrachteten Stimulationselektrode bietet den Vorteil einer Anpassung an das bei vielen Patienten vorliegende Empfinden, wonach bei niedrigen Werten des Stimulationsstromes Änderungen desselben stark wahrgenommen werden, während bei hohen Werten des Stimulationsstromes Änderungen desselben in deutlich geringerem Maß wahrgenommen werden.

Eine Stimulationsbehandlung erstreckt sich in der Regel über längere Zeiträume, wobei Zeitintervalle, in denen ein Stromfluss zu den Stimulationselektroden erfolgt mit Zeitintervallen abwechseln, in denen der Stromfluss unterbrochen ist. Meist werden dabei in abwechselnder Reihenfolge kurze Stromflusszeitintervalle und kurze Inaktiv-Zeitintervalle und nach einer größeren Anzahl solcher Zyklen längere Pausen, z.B. mit einer Länge von 1 Stunde, vorgesehen.

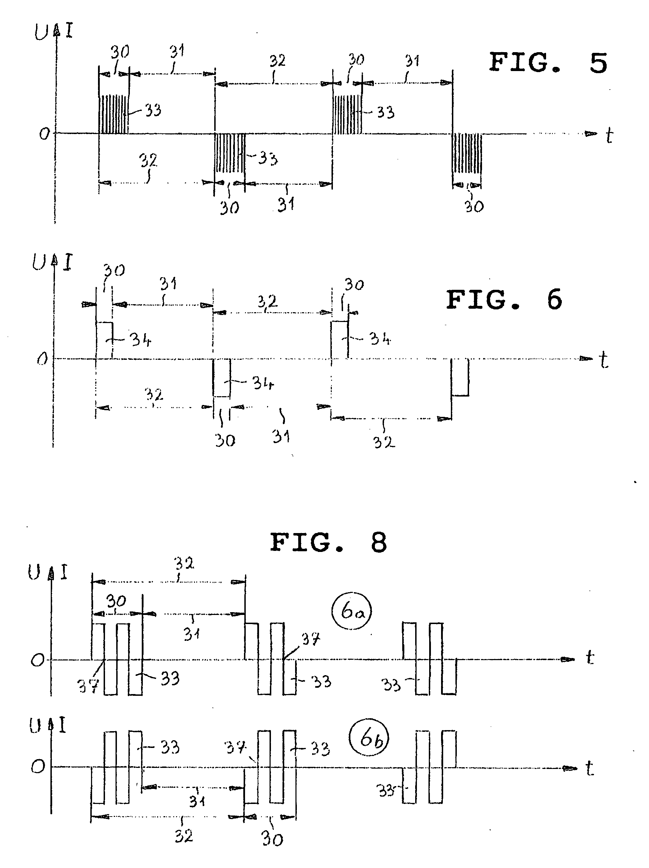

Fig. 5 zeigt den zeitlichen Ablauf einer solchen Stimulation, wobei Stromflusszeitintervalle 30 in zyklischer Aufeinanderfolge mit Inaktiv-Zeitintervallen 31 abwechseln und jeweils ein Stromflusszeitintervall 30 und ein Inaktiv-Zeitintervall 31 zusammen einen Stimulationszyklus 32 bilden. In den Stromflusszeitintervallen 30 hat der Behandlungsstrom die Form eines Pakets aufeinander folgender Impulse 33, die z.B. je einige Millisekunden dauern. Bei jedem Stromflusszeitintervall 30, bei dem in Fig. 5 dargestellten Fall am Anfang desselben, erfolgt ein Wechsel der Polarität des Behandlungsstromes.

Fig. 6 zeigt eine Variante zu dem in Fig. 5 dargestellten zeitlichen Ablauf einer Stimulationsbehandlung, wobei in dem in Fig. 6 dargestellten Fall der in den Stromflusszeitintervallen 30 fließende Behandlungsstrom in jedem solchen Zeitintervall 30 die Form eines Einzelimpulses 34 hat. Am Anfang jedes Stromflussintervalls 30 findet, analog wie bei dem in Fig. 5 dargestellten Ablauf, ein Polaritätswechsel des Behandlungsstromes statt.

In den Fig. 5 und 6 sind die Stimulationsimpulse mit einem konstanten Spannungswert dargestellt, entsprechend einem Verhalten der in den Ausgangskanälen des erfindungsgemäßen Gerätes vorgesehenen Verstärker als Konstantspannungsquelle.

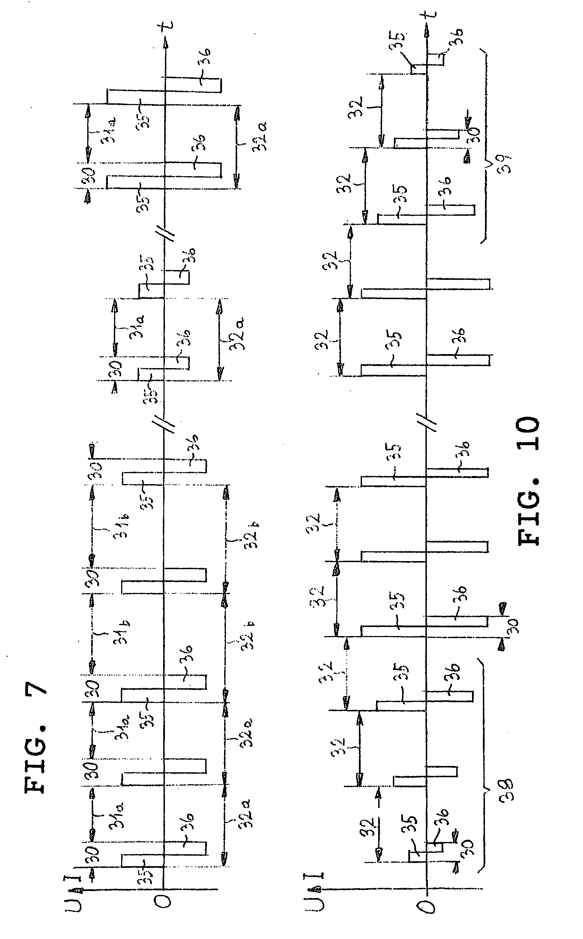

Fig. 7 zeigt den zeitlichen Ablauf einer weiteren Form der Stimulation. Der in den Stromflusszeitintervallen 30 fließende Behandlungsstrom hat dabei die Form von Doppelimpulsen 35, 36, deren Polarität einander entgegengesetzt ist, wobei der Polaritätswechsel im Verlauf der Stromflusszeitintervalle 30 erfolgt. Es ist bei dieser Ausführungsform, die insbesondere auf eine adaptive Regelung des Behandlungsstromes abgestellt ist, vorgesehen, dass die Intensität des Behandlungsstromes in Anpassung an die jeweils vorliegenden Gegebenheiten variiert wird. Es werden dabei einerseits die Inaktiv-Zeitintervalle der Stimulationszyklen variiert, wodurch sich auch die Länge der Stimulationszyklen verändert, wobei z.B. die Inaktiv-Zeitintervalle 31b länger als das Inaktiv-Zeitintervall 31a sind und korrespondierend die Stimulationszyklen 32b länger als der Stimulationszyklus 32a. Es ergibt sich so eine Variation des über des Zeitdauer eines Stimulationszyklus bestimmten Mittelwertes der Stromstärke durch die Variation der Zeitdauer des Stimulationszyklus. Die Stromstärke ist dabei unter außer Acht lassen des Polaritätswechsels anhand der Summe der einzelnen Impulse 35, 36 in Rechnung zu stellen. Es ist weiters bei dem in Fig. 7 dargestellten Ablauf eines Stimulationsvorganges eine Variation der Amplitude der Behandlungsströme vorgesehen.

Fig. 8 zeigt im zeitlichen Verlauf die Behandlungsströme, welche an zwei Stimulationselektroden vorliegen, die je einem Ausgangskanal eines erfindungsgemäß ausgebildeten Gerätes zugeordnet sind. Die Behandlungsströme sind dabei in den Stromflusszeitintervallen 30 je durch ein Paket aufeinander folgender Impulse 33 gebildet, wobei jeweils beim Übergang von einem Paket dieser Impulse 33 zum nächsten Paket dieser Impulse ein Polaritätswechsel 37 erfolgt. In Bezug auf eine mit dem Behandlungsstromkreisen in Verbindung stehende Bezugselektrode weist der der Stimulationselektrode 6a zugeführte Behandlungsstrom in den Stromflusszeitintervallen 30 eine gegenüber dem der Stimulationselektrode 6b zugeführten Behandlungsstrom unterschiedliche Polarität auf. Da weiter die Intensitäten der Impulse 33 des der Stimulationselektrode 6a zugeführten Behandlungsstromes den Intensitäten der Impulse 33 des der Stimulationselektrode 6b zugeführten Behandlungsstromes gleich sind und durch die im jeweils betrachteten Zeitpunkt voneinander unterschiedliche Polarität einander kompensieren, ergibt sich, dass hierbei über eine mit den Behandlungsstromkreisen in Verbindung stehende Bezugselektrode kein Behandlungsstrom fließt. Bei ungleicher Intensität der Impulse der den bei den Stimulationselektroden 6a, 6b zugeführten Behandlungsströme ergibt sich nur eine teilweise Kompensation und es findet ein, wenn auch verminderter, Stromfluss über eine solche Bezugselektrode statt.

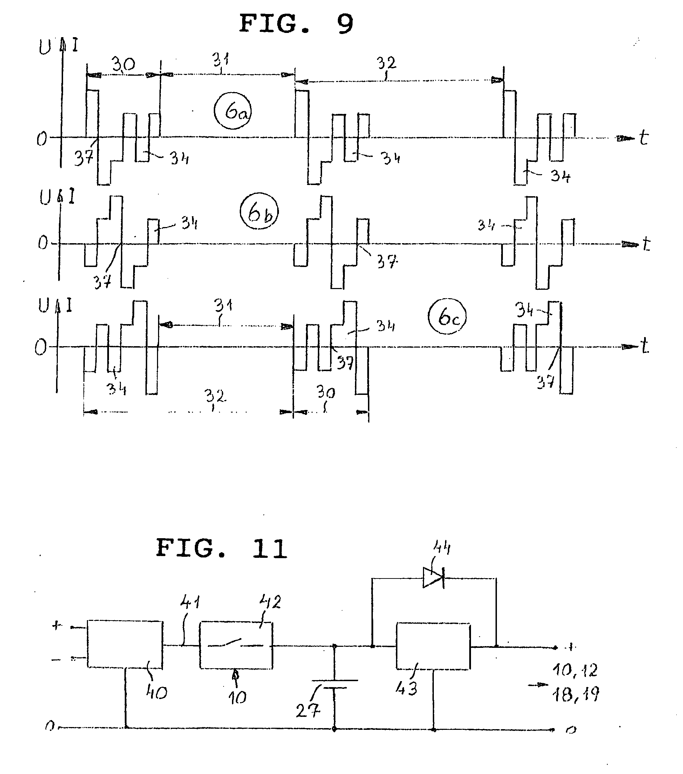

Fig. 9 zeigt den zeitlichen Ablauf von Behandlungsströmen, welche drei Stimulationselektroden 6a, 6b, 6c zugeführt werden, wobei diese Stimulationselektroden ihrerseits je einem Ausgangskanal eines erfindungsgemäß ausgebildeten Gerätes zugeordnet sind. Die den einzelnen Stimulationselektroden 6a, 6b, 6c zugeführten Behandlungsströme sind in jedem Stromflusszeitintervall 30 durch einen Einzelimpuls 34 gebildet, wobei in jedem dieser Einzelimpulse zeitlich aufeinander folgend mehrere Intensitätsveränderungen und Polaritätswechsel 37 vorgesehen sind. Die Intensitätsveränderungen der Behandlungsströme erfolgen dabei stufenweise. Es gilt dabei, dass die an einer jeweils betrachteten Stimulationselektrode erfolgenden Intensitätsveränderungen gegenläufig zu den an einer oder mehreren anderen jeweils betrachteten Stimulationselektroden erfolgenden Intensitätsveränderungen verlaufen. Durch gegenseitige Abstimmung des Ausmaßes der Intensitätsveränderungen wird erzielt, dass in jedem jeweils betrachteten Zeitpunkt im Rahmen der Stromflusszeitintervalle der jeder einzelnen Stimulationselektrode 6a, 6b, 6c zugeführte Behandlungsstrom hinsichtlich Intensität und Polarität durch die den jeweils beiden anderen Stimulationselektroden 6a, 6b, 6c zugeführten Behandlungsströme kompensiert ist. Es ergibt sich solcher Art, dass kein Behandlungsstrom über eine am Gerät vorgesehene Bezugselektrode fließt. Auch allfällig auftretende Kontaktunsicherheiten an einer der Stimulationselektroden werden weitgehend selbsttätig ausgeglichen, wobei auch in einem solchen Fall ein allfällig über eine Bezugselektrode fließender Ausgleichsstrom gering bleibt.

Bei dem in Fig. 10 dargestellten Ablauf eines einer Stimulationselektrode zugeführten Behandlungsstromes wird am Beginn 38 einer durch eine Vielzahl von aufeinander folgenden Stimulationszyklen 32 gebildeten Stimulationszyklenfolge, während mehrerer aufeinander folgender Stimulationszyklen die Intensität des Behandlungsstromes, der einer jeweils betrachteten Stimulationselektrode 6a, 6b, 6c zugeführt wird, als Mittelwert über das jeweilige Stromflusszeitintervall 30 betrachtet, fortlaufend von jeweils einem Stimulationszyklus 32 zum darauf folgenden Stimulationszyklus erhöht und danach während einer Vielzahl aufeinander folgender Stimulationszyklen auf dem zuvor erreichten Pegel der Intensität gehalten. Am Ende 39 der Stimulationszyklenfolge wird die Intensität des einer jeweils betrachteten Stimulationselektrode zugeführten Behandlungsstromes fortlaufend von jeweils einem Stimulationszyklus zum darauf folgenden Stimulationszyklus vermindert.

Die in den Ausführungsbeispielen der Zeitabläufe von zur Stimulation vorgesehenen Behandlungsströmen vorliegenden Merkmale sind nicht nur allein im Zusammenhang des jeweiligen Ausführungsbeispiels zu sehen, sondern im Rahmen der Erfindung auch miteinander kombinierbar. Insbesondere sind die der besseren Übersicht wegen für einen Ausgangskanal dargestellten Zeitabläufe, wie sie die Figuren 5, 6, 7 und 10 zeigen, dem Erfindungskonzept folgend, auf mehrere Ausgangskanäle anwendbar, wobei auch hinsichtlich der Parameter der vorzusehenden Stimulationsimpulse überall verschiedene Möglichkeiten bestehen. So können in den verschiedenen Ausführungsformen wahlweise Pakete aufeinander folgender Impulse oder Einzelimpulse vorgesehen werden, wobei verschiedene Varianten von Polaritätswechseln und Intensitätsveränderungen gangbar sind. Es kommt neben der hier angesprochenen Zufuhr unterschiedlicher Ströme an die verschiedenen Stimulationselektroden gegebenenfalls auch eine gleichlaufende Speisung dieser Elektroden in Frage.

Bei der in Fig. 11 dargestellten Ausführungsform einer in einem erfindungsgemäß ausgebildetem Gerät vorgesehenen Betriebsspannungsversorgungsschaltung ist zur Speisung der in den Ausgangskanälen 9a, 9b, 9c vorgesehenen Verstärker 15a, 15b, 15c ein Spannungswandler 40 mit bipolarem Ausgang vorgesehen, der seinerseits von der im Gerät vorgesehenen Batterie 27 gespeist wird. In die von der Batterie 27 zum Spannungswandler 40 führende speisende Verbindung 41 ist eine Schalteinrichtung 42 eingefügt, welche unter Steuerung von der Mikrocomputerschaltung 10 die Speisung des Spannungswandlers 40 sowohl während in den Behandlungsstromkreisen vorliegender Stromflusspausen als auch bei Überschreitung eines vorgegebenen Grenzwertes des der Batterie 27 vom Spannungswandler 40 entnommenen Stromes unterbricht. Zur Speisung der Mikrocomputerschaltung 10, des Digital-Analog-Wandlers 12 und allfällig vorgesehener Messsignalschaltungen 18a, 18b, 18c, 19a, 19b, 19c ist ein an die Batterie 27 angeschlossener Spannungswandler 43 mit unipolarem Ausgang vorgesehen, der mit einer Schottky-Diode 44 überbrückt ist und nur bei Absinken der Batteriespannung aktiviert wird und an seinem Ausgang eine der Batterie-Sollspannung entsprechende Spannung abgibt.

Die Übertragungseinrichtung 29 ist vorzugsweise sowohl zur Eingabe von zu speichernden Parametern der für die jeweilige Behandlung vorgesehene Behandlungsströme als auch zur Übermittlung von im Speicher 11 der Mikrocomputerschaltung 10 gespeicherten Parametern der für die jeweilige Behandlung vorgesehenen Behandlungsströme an ein externes Steuer- und Kontrollgerät eingerichtet.

**Patentansprüche**

1. Gerät (1) zur Punktual-Stimulation von im Bereich der Ohren liegenden Endigungen von zu Hirnstammkernen führenden Nerven, welches Gerät (1) einen batteriegespeisten Behandlungsstromgenerator (3) aufweist, der in einem im Ohrbereich zu tragenden Gehäuse (4) angeordnet ist und der mit einer niederfrequenten Behandlungsstrom formenden Elektronikschaltung (10, 12) versehen ist, und welches Gerät (1) mindestens zwei vom Behandlungsstromgenerator (3) ausgehende flexible Leitungen (5a, 5b, 5c) zur Verbindung mit je einer an einer Nervenendigung zu positionierenden Stimulations-Nadelelektrode (6a, 6b, 6c) aufweist, wobei der Behandlungsstromgenerator (3) mehrere analog arbeitende Ausgangskanäle (9a, 9b, 9c) aufweist, und jeder dieser Ausgangskanäle einer eigenen Stimulationselektrode (6a, 6b, 6c) zugeordnet ist und diese analog arbeitenden Ausgangskanäle zur Steuerung des Behandlungsstromes eingangsseitig je für sich an eine Mikrocomputerschaltung (10) angeschlossen sind, in der Parameterwerte der für die jeweilige Behandlung vorgesehenen Behandlungsströme zur fortlaufenden Auslesung in einem Speicher (11) speicherbar sind, **dadurch gekennzeichnet, dass** in der Mikrocomputerschaltung (10) ein durch externe Betätigung wahlweise aktivierbarer Adaptivstrom-Steuerkreis (22) vorgesehen ist, der mit einem internen Speicher (11) für Behandlungsstromparameter wirkverbunden ist und über einen Digital-Analog-Wandler (12), der an einen Digitalanschluss (13) der Mikrocomputerschaltung (10) angeschlossen ist, mindestens einem in einem Ausgangskanal (9a, 9b, 9c) vorgesehenen, für bipolare Ausgangssignale ausgebildeten Verstärker (15a, 15b, 15c) ein Steuersignal zuführt, das in dem mit einer Stimulationselektrode (6a, 6b, 6c) verbundenen Ausgangskreis dieses Verstärkers (15a, 15b, 15c) ein dem in diesem Ausgangskreis jeweils vorliegenden Widerstandswert adaptiv folgendes Verhalten von Stromstärke und Stimulations-spannung entsprechend den Beziehungen

$$I = k \cdot R$$

$$U = k \cdot R^2$$

herbeiführt, wobei k eine wählbare Konstante ist.

2. Gerät (1) nach Anspruch 1, **dadurch gekennzeichnet, dass** in den Ausgangskanälen (9a, 9b, 9c) in die zu den Stimulationselektroden (6a, 6b, 6c) führenden Stromwege (16a, 16b, 16c) Messwiderstände (17a, 17b, 17c) zur Strommessung eingefügt sind und an die einzelnen Messwiderstände (17a, 17b, 17c) jeweils ein Messverstärker (18a, 18b, 18c) angeschlossen ist, dessen Ausgangssignal ein Maß für die Stromstärke des Behandlungsstromes ist und der Mikrocomputerschaltung (10) zugeleitet wird, und an mindestens einem Ausgangskanal (9a, 9b, 9c) eine Spannungsmessschaltung (19a, 19b, 19c) vorgesehen ist, welche an die Mikrocomputerschaltung (10) ein Messsignal abgibt, das der Spannung entspricht, die zwischen der dem betreffenden Ausgangskanal zugeordneten Stimulationselektrode (6a, 6b, 6c) und einem im Behandlungsstromgenerator (3) befindlichen Bezugspotential-Punkt (0), der mit einer Bezugselektrode (7) verbunden ist, vorliegt.

3. Gerät (1) nach Anspruch 1 oder 2, **dadurch gekennzeichnet, dass** in den Adaptivstrom-Steuerkreis (22) ein Adaptivstrom-Regelkreis (25) eingebunden ist, dem ein aus dem Messsignal der Spannungsmessschaltung (19a, 19b, 19c) und dem Ausgangssignal des Messverstärkers (18a, 18b, 18c) der Strommessung gewonnener Widerstandsmesswert als Istsignal zugeführt wird.

4. Gerät (1) nach Anspruch 1, **dadurch gekennzeichnet, dass** der in der Mikrocomputerschaltung (10) vorgesehene Steuerkreis (20, 21, 22) den in den Ausgangskanälen vorgesehenen Verstärkern (15a, 15b, 15c) ein Steuersignal zuführt, das in zyklischer Aufeinanderfolge Zeitintervalle (30), in denen ein Stromfluss zu den Stimulationselektroden erfolgt, und Zeitintervalle (31), in denen die Ausgangskanäle inaktiv sind, bildet, wobei jeweils ein Stromflusszeitintervall (30) und ein Inaktiv-Zeitintervall (31) zusammen einen Stimulationszyklus (32) bilden, und das eine Variation des über die Zeitdauer eines Stimulationszyklus (32) bestimmten Mittelwertes der Stromstärke durch Variation dieser Zeitdauer durch das Steuersignal herbeiführt.

5. Gerät (1) nach Anspruch 1, **dadurch gekennzeichnet, dass** in der Mikrocomputerschaltung (10) ein durch externe Betätigung wahlweise aktivierbarer Konstantspannungs-Steuerkreis (20) vorgesehen ist, der mit einem internen Speicher (11) für Behandlungsstromparameter wirkverbunden ist und über einen Digital-Analog-Wandler (12) mindestens einem in einem Ausgangskanal (9a, 9b, 9c) vorgesehenen Verstärker (15a, 15b, 15c) ein Steuersignal zuführt, das in dem mit einer Stimulationselektrode (6a, 6b, 6c) verbundenen Ausgangskreis dieses Verstärkers (15a, 15b, 15c) das Verhalten einer hinsichtlich des Spannungswertes einstellbaren Konstantspannungsquelle herbeiführt.

6. Gerät (1) nach Anspruch 2 und 5, **dadurch gekennzeichnet, dass** in den Konstantspannungs-Steuerkreis (20) ein Konstantspannungs-Regelkreis (23) eingebunden ist, dem das Messsignal der betreffenden Spannungsmessschaltung (19a, 19b, 19c) als Istsignal zugeführt wird.

7. Gerät (1) nach Anspruch 1 und 2, **dadurch gekennzeichnet, dass** in der Mikrocomputerschaltung (10) ein durch externe Betätigung wahlweise aktivierbarer Konstantstrom-Steuerkreis (21) vorgesehen ist, der mit einem internen Speicher (11) für Behandlungsstromparameter wirkverbunden ist und über einen Digital-Analog-Wandler (12) mindestens einem in einem Ausgangskanal (9a, 9b, 9c) vorgesehenen Verstärker (15a, 15b, 15c) ein Steuersignal zuführt, das in dem mit einer Stimulationselektrode (6a, 6b, 6c) verbundenen Ausgangskreis dieses Verstärkers (15a, 15b, 15c) das Verhalten einer hinsichtlich der Stromstärke einstellbaren Konstantstromquelle herbeiführt, und in den Konstantstrom-Steuerkreis (21) ein Konstantstrom-Regelkreis (24) eingebunden ist, dem das Ausgangssignal des Messverstärkers (18a, 18b, 18c) als Istsignal zugeführt wird.

8. Gerät (1) nach einem der Ansprüche 1 bis 7, **dadurch gekennzeichnet, dass** der in der Mikrocomputerschaltung (10) vorgesehene Steuerkreis (20, 21, 22) den in den Ausgangskanälen vorgesehenen Verstärkern (15a, 15b, 15c) ein Steuersignal zuführt, das in zyklischer Aufeinanderfolge Zeitintervalle (30), in denen ein Stromfluss zu den Stimulationselektroden erfolgt, und Zeitintervalle (31), in denen die Ausgangskanäle inaktiv sind, bildet und im Verlauf jedes Stromflusszeitintervalles (30) einen Polaritätswechsel herbeiführt, wobei jeweils ein Stromflusszeitintervall (30) und ein Inaktiv-Zeitintervall (31) zusammen einen Stimulationszyklus (32) bilden und in den Stromflusszeitintervallen (30) ein Behandlungsstrom in Form eines Paketes aufeinander folgender Impulse (33) oder in Form eines Einzelimpulses (34) fließt.

9. Gerät (1) nach einem der vorhergehenden Ansprüche, **dadurch gekennzeichnet, dass** in der Mikrocomputerschaltung (10) ein Steuerkreis (20, 21, 22) vorgesehen ist, der den in den Ausgangskanälen vorgesehenen Verstärkern (15a, 15b, 15c) ein Steuersignal zuführt, das in zyklischer Aufeinanderfolge Zeitintervalle (30), in denen ein Stromfluss zu den Stimulationselektroden erfolgt, und Zeitintervalle (31), in denen die Ausgangskanäle inaktiv sind, bildet, wobei jeweils ein Stromflusszeitintervall (30) und ein Inaktiv-Zeitintervall (31) zusammen einen Stimulationszyklus (32) bilden, und der im Zuge der einzelnen Stromflusszeitintervalle eine oder mehrere Veränderungen der Intensität der den einzelnen Stimulationselektroden zugeführten Behandlungsströme herbeiführt, wobei die an einer jeweils betrachteten Stimulationselektrode (6a, 6b, 6c) erfolgenden Intensitätsveränderungen gegenläufig zu den an einer oder mehreren anderen jeweils betrachteten Stimulationselektrode (n) erfolgenden Intensitätsveränderungen verlaufen.

10. Gerät (1) nach Anspruch 9, **dadurch gekennzeichnet, dass** drei je einer eigenen Stimulationselektrode zugeordnete Ausgangskanäle vorgesehen sind und in den Stromflusszeitintervallen in jedem jeweils betrachteten Zeitpunkt der jeder einzelnen Stimulationselektrode (6a, 6b, 6c) zugeführte Behandlungsstrom hinsichtlich Intensität und Polarität durch die den jeweils beiden anderen Stimulationselektroden (6a, 6b, 6c) zugeführten Behandlungsströme kompensiert ist.

11. Gerät (1) nach Anspruch 9 oder 10, **dadurch gekennzeichnet, dass** die den einzelnen Stimulationselektroden (6a, 6b, 6c) zugeführten Behandlungsströme in jedem Stromflusszeitintervall (30) durch einen Einzelimpuls (34) gebildet sind und in jedem dieser Einzelimpulse zeitlich aufeinander folgend mehrere Intensitätsveränderungen und mindestens ein Polaritätswechsel (37) vorgesehen sind.

12. Gerät (1) nach einem der Ansprüche 1 bis 11, **dadurch gekennzeichnet, dass** die in den einzelnen Stromflusszeitintervallen (30) vorgesehene ein- oder mehrmalige Veränderung der Behandlungsstromintensität stufenweise erfolgt.

13. Gerät (1) nach einem der Ansprüche 8 bis 12, **dadurch gekennzeichnet, dass** der in der Mikrocomputerschaltung (10) vorgesehene Steuerkreis (20, 21, 22) den in den Ausgangskanälen vorgesehenen Verstärkern (15a, 15b, 15c) ein Steuersignal zuführt, das in zyklischer Aufeinanderfolge Zeitintervalle (30), in denen ein Stromfluss zu den Stimulationselektroden erfolgt, und Zeitintervalle (31), in denen die Ausgangskanäle inaktiv sind, bildet, wobei jeweils ein Stromflusszeitintervall (30) und ein Inaktiv-Zeitintervall (31) zusammen einen Stimulationszyklus (32) bilden, und am Beginn (38) einer durch eine Vielzahl von aufeinander folgenden Stimulationszyklen (32) gebildeten Stimulationszyklenfolge während mehrerer aufeinander folgender Stimulationszyklen die Intensität des einer jeweils betrachteten Stimulationselektrode (6a, 6b, 6c) zugeführten Behandlungsstromes, als Mittelwert über das jeweilige Stromflusszeitintervall (30) betrachtet, fortlaufend von jeweils einem Stimulationszyklus (32) zum darauf folgenden Stimulationszyklus erhöht und danach während einer Vielzahl aufeinander folgender Stimulationszyklen in deren Stromflusszeitintervallen den zuvor erreichten Pegel der Intensität der Behandlungsströme aufrecht erhält.

14. Gerät (1) nach Anspruch 13, **dadurch gekennzeichnet, dass** am Ende (39) der Stimulationszyklenfolge der Steuerkreis den in den Ausgangskanälen vorgesehenen Verstärkern ein Steuersignal zuführt, das die Intensität des einer jeweils betrachteten Stimulationselektrode (6a, 6b, 6c) zugeführten Behandlungsstromes, als Mittelwert über das jeweilige Stromflusszeitintervall betrachtet, fortlaufend von jeweils einem Stimulationszyklus (32) zum darauf folgenden Stimulationszyklus vermindert.

15. Gerät (1) nach einem der Ansprüche 1 bis 14, **dadurch gekennzeichnet, dass** zur Speisung der in den Ausgangskanälen (9a, 9b, 9c) vorgesehenen Verstärker (15a, 15b, 15c) ein Spannungswandler (40) mit bipolarem Ausgang vorgesehen ist, der seinerseits von der im Gerät vorgesehenen Batterie (27) gespeist wird.

16. Gerät (1) nach Anspruch 15, **dadurch gekennzeichnet, dass** in die von der Batterie (27) zum Spannungswandler (40) führende speisende Verbindung (41) eine Schalteinrichtung (42) eingefügt ist, welche die Speisung des Spannungswandlers (40) sowohl während in den Behandlungsstromkreisen vorliegender Stromflusspausen als auch bei Überschreitung eines vorgegebenen Grenzwertes des der Batterie (27) vom Spannungswandler (40) entnommenen Stromes unterbricht.

17. Gerät (1) nach einem der Ansprüche 1 bis 16, **dadurch gekennzeichnet, dass** zur Speisung der Mikrocomputerschaltung (10), des Digital-Analog-Wandlers (12) und von im Gerät (1) vorgesehenen Messsignalschaltungen (18a, 18b, 18c, 19a, 19b, 19c) ein an die Batterie (27) angeschlossener Spannungswandler (43) mit unipolarem Ausgang vorgesehen ist, der mit einer Schottky-Diode (44) überbrückt ist und nur bei Absinken der Batteriespannung aktiviert wird und an seinem Ausgang eine der Batterie-Sollspannung entsprechende Spannung abgibt.

## Claims

1. A device (1) for the punctual stimulation of endings of nerves located in the region of the ears leading to brain stem nuclei, which device (1) comprises a battery-fed treatment current generator (3) that is arranged in a housing (4) to be worn in a region of an ear and that is provided with an electronic circuit (10, 12) forming low-frequency treatment current, and which device (1) comprises at least two flexible wires (5a, 5b, 5c) starting at the treatment current generator (3), each of which is to be connected to a stimulation needle electrode (6a, 6b, 6c) to be positioned at a nerve ending, wherein the treatment current generator (3) has several analogously operating output channels (9a, 9b, 9c) and each of these output channels is associated to a stimulation electrode (6a, 6b, 6c) of its own, and for controlling the treatment current each of these analogously operating output channels is separately connected to a microcomputer circuit (10) on the input side, in which parameter values of the treatment currents intended for the respective treatments may be stored in a memory (11) for continuously reading them out, **characterised in that** in the microcomputer circuit (10) an adaptive current control circuit (22) to be selectively activated by external actuation is provided, which is effectively connected to an internal memory (11) for treatment current parameters and supplies to at least one amplifier (15a, 15b, 15c) provided in an output channel (9a, 9b, 9c) and designed for bipolar output signals, via a digital-analog converter (12) connected to a digital port (13) of the microcomputer circuit (10), a control signal which effects, in the output circuit of this amplifier (15a, 15b, 15c) connected to a stimulation electrode (6a, 6b, 6c), a behaviour of amperage and stimulation voltage adaptively following the respective resistance value present in this output circuit and corresponding to the relations

$$I = k \cdot R$$

$$U = k \cdot R^2$$

wherein k is a selectable constant.

2. The device (1) of claim 1, **characterised in that** in the output channels (9a, 9b, 9c) measuring resistors (17a, 17b, 17c) for measuring current are included in the current paths (16a, 16b, 16c) leading to the stimulation electrodes (6a, 6b, 6c) and a measuring amplifier (18a, 18b, 18c) is connected to each of the individual measuring resistors (17a, 17b, 17c), the output signal of which is a measure for the amperage of the treatment current and is supplied to the the microcomputer circuit (10), and a voltage measuring circuit (19a, 19b, 19c) is provided at at least one output channel (9a, 9b, 9c), outputting a measuring signal to the microcomputer circuit (10), which corresponds to the voltage present between the stimulation electrode (6a, 6b, 6c) associated to the respective output channel and a reference potential point (0) located in the treatment current generator (3), which is connected to a reference electrode (7).

3. The device (1) of claim 1 or 2, **characterised in that** in the adaptive current control circuit (22) an adaptive current regulatory circuit (25) is incorporated, to which a resistance measuring value obtained from the measuring signal of the voltage measuring circuit (19a, 19b, 19c) and the output signal of the measuring amplifier (18a, 18b, 18c) of the current measuring is supplied as an actual signal.

4. The device (1) of claim 1, **characterised in that** the control circuit (20, 21, 22) provided in the microcomputer circuit (10) supplies a control signal to the amplifiers (15a, 15b, 15c) provided in the output channels, which forms, in a cyclic sequence, time intervals (30) in which a current flow to the stimulation electrodes occurs and time intervals (31) in which the output channels are inactive, wherein a respective current flow time interval (30) and a respective inactive time interval (31) together form a stimulation cycle (32), and which effects a variation in the mean value of the amperage determined over the duration of a stimulation cycle (32) via a variation of this duration by the control signal.

5. The device (1) of claim 1, **characterised in that** in the microcomputer circuit (10) a constant voltage control circuit (20) to be selectively activated by external actuation is provided, which is effectively connected to an internal memory (11) for treatment current parameters and supplies to at least one amplifier (15a, 15b, 15c) provided in an output channel (9a, 9b, 9c), via a digital-to-analogue converter (12), a control signal which effects, in the output circuit of this amplifier (15a, 15b, 15c) connected to a stimulation electrode (6a, 6b, 6c), the behaviour of a constant voltage source that is adjustable in regard to the voltage value.

6. The device (1) of claims 2 and 5, **characterised in that** in the constant voltage control circuit (20) a constant voltage regulatory circuit (23) is incorporated, to which the measuring signal of the respective voltage measuring circuit (19a, 19b, 19c) is supplied as an actual signal.

7. The device (1) of claims 1 and 2, **characterised in that** in the microcomputer circuit (10) a constant current control circuit (21) to be selectively activated by external actuation is provided, which is effectively connected to an internal memory (11) for treatment current parameters and supplies to at least one amplifier (15a, 15b, 15c) provided in an output channel (9a, 9b, 9c), via a digital-analog converter (12), a control signal which effects, in the output circuit of this amplifier (15a, 15b, 15c) connected to a stimulation electrode (6a, 6b, 6c), the behaviour of a constant current source that is adjustable in regard to the amperage, and in the constant current control circuit (21) a constant current regulatory circuit (24) is incorporated, to which the output signal of the measuring amplifier (18a, 18b, 18c) is supplied as an actual signal.

8. The device (1) of any one of claims 1 to 7, **characterised in that** the control circuit (20, 21, 22) provided in the microcomputer circuit (10) supplies a control signal to the amplifiers (15a, 15b, 15c) provided in the output channels, which forms, in a cyclic sequence, time intervals (30) in which a current flow to the stimulation electrodes occurs and time intervals (31) in which the output channels are inactive, and effects a polarity reversal in the course of each current flow time interval (30), wherein a respective current flow time interval (30) and a respective inactive time interval (31) together form a stimulation cycle (32), and in the current flow time intervals (30) a treatment current flows in the form of a package of sequential pulses (33) or in the form of a single pulse (34).

9. The device (1) of any one of the preceding claims, **characterised in that** in the microcomputer circuit (10) a control circuit (20, 21, 22) is provided, which supplies a control signal to the amplifiers (15a, 15b, 15c) provided in the output channels, which forms, in a cyclic sequence, time intervals (30) in which a current flow to the stimulation electrodes occurs and time intervals (31) in which the output channels are inactive, wherein a respective current flow time interval (30) and a respective inactive time interval (31) together form a stimulation cycle (32), and which effects, in the course of the individual current flow time intervals, one or more intensity changes of the treatment currents supplied to the individual stimulation electrodes, wherein the intensity changes occurring at a respective stimulation electrode (6a, 6b, 6c) that is being observed occur in the opposite direction to the intensity changes occurring at one or more other respective stimulation electrodes (n) that are being observed.

10. The device (1) of claim 9, **characterised in that** three output channels, each associated to a stimulation electrode of its own, are provided, and the treatment current supplied to each individual stimulation electrode (6a, 6b, 6c) in the current flow time intervals at each respective point in time under observation is compensated by the treatment currents supplied to the respective two other stimulation electrodes (6a, 6b, 6c) in regard to intensity and polarity.

**11.** The device (1) of claim 9 or 10, **characterised in that** the treatment currents supplied to the individual stimulation electrodes (6a, 6b, 6c) in each current flow time interval (30) are formed by a single pulse (34), and in each of these single pulses several temporarily sequential intensity changes and at least one polarity reversal (37) are provided.

**12.** The device (1) of any one of claims 1 to 11, **characterised in that** the single- or multiple-time intensity change of the treatment current provided in the individual current flow time intervals (30) occurs in a step-like manner.

**13.** The device (1) of any one of claims 8 to 12, **characterised in that** the control circuit (20, 21, 22) provided in the microcomputer circuit (10) supplies a control signal to the amplifiers (15a, 15b, 15c) provided in the output channels, which forms, in a cyclic sequence, time intervals (30) in which a current flow to the stimulation electrodes occurs and time intervals (31) in which the output channels are inactive, wherein a respective current flow time interval (30) and a respective inactive time interval (31) together form a stimulation cycle (32), and at the start (38) of a stimulation cycle sequence formed by a plurality of sequential stimulation cycles (32) the intensity of the treatment current supplied to a respective stimulation electrode (6a, 6b, 6c) that is being observed, considered as a mean value over the respective current flow time interval (30), is increased continually from one respective stimulation cycle (32) to the following stimulation cycle during several sequential stimulation cycles and then maintains the previously obtained level of intensity of the treatment currents during a plurality of sequential stimulation cycles in their current flow time intervals.

**14.** The device (1) of claim 13, **characterised in that** at the end (39) of the stimulation cycle sequence the control circuit supplies a control signal to the amplifiers provided in the output channels, which decreases the intensity of the treatment current supplied to a respective stimulation electrode (6a, 6b, 6c) that is being observed, considered as a mean value over the respective current flow time interval, continually from one respective stimulation cycle (32) to the following stimulation cycle.

**15.** The device (1) of any one of claims 1 to 14, **characterised in that** for feeding the amplifiers (15a, 15b, 15c) provided in the output channels (9a, 9b, 9c) a voltage converter (40) having a bipolar output is provided, which is in turn fed by the battery (27) provided in the device.

**16.** The device (1) of claim 15, **characterised in that** a switching device (42) is included in the feeding connection (41) leading from the battery (27) to the voltage converter (40), which interrupts the feeding of the voltage converter (40) both during the current flow pauses present in the treatment current circuits and when exceeding a given threshold of the current consumed from the battery (27) by the voltage converter (40).

**17.** The device (1) of any one of claims 1 to 16, **characterised in that** for supplying the microcomputer circuit (10), the digital-analog converter (12) and measuring signal circuits (18a, 18b, 18c, 19a, 19b, 19c) provided in the device (1) a voltage converter (43) connected to the battery (27) having an unipolar output is provided, which is bridged by a Schottky diode (44) and is activated to output a voltage corresponding to the battery's nominal voltage at its output only when the voltage of the battery decreases.

**Revendications**

**1.** Appareil (1) pour la stimulation ponctuelle de terminaisons nerveuses situées au niveau des oreilles conduisant à des noyaux du tronc cérébral, cet appareil (1) comprenant un générateur de courant de traitement (3) alimenté par batterie, qui est disposé dans un boîtier (4) à porter au niveau de l'oreille et qui est muni d'un circuit électronique (10, 12) générant un courant de traitement à basse fréquence, et cet appareil (1) comprenant au moins deux câbles flexibles (5a, 5b, 5c) provenant du générateur de courant de traitement (3), destinés chacun à être relié à une électrode aiguille de stimulation (6a, 6b, 6c) à positionner sur une terminaison nerveuse, le générateur de courant de traitement (3) comprenant plusieurs canaux de sortie (9a, 9b, 9c) fonctionnant de manière analogique et chacun de ces canaux de sortie correspondant à sa propre électrode de stimulation (6a, 6b, 6c) et ces canaux de sortie fonctionnant de manière analogique étant branchés chacun, pour le contrôle du courant de traitement, du côté de l'entrée, à un circuit de micro-ordinateur (10), dans lequel les valeurs des paramètres des courants de traitement prévus pour le traitement concerné peuvent être enregistrées dans une mémoire (11) pour une lecture en continu, **caractérisé en ce que**, dans le circuit de micro-ordinateur (10), se trouve un circuit de commande à courant adaptatif (22) qui peut être activé sélectivement par actionnement externe et qui est relié de manière fonctionnelle avec une mémoire interne (11) pour les paramètres du courant de traitement et introduit, par l'intermédiaire d'un convertisseur numérique-analogique (12), qui est branché à une connexion numérique (13) du circuit de micro-ordinateur (10), dans au moins un amplificateur (15a, 15b, 15c) disposé dans un canal de sortie (9a, 9b, 9c) et conçu pour des signaux de sortie bipolaires, un signal de commande qui provoque, dans le circuit de sortie de cet amplificateur (15a, 15b, 15c), relié à une électrode de stimulation (6a, 6b, 6c), un comportement de l'intensité et de la tension de stimulation suivant de manière adaptative la valeur de résistance se trouvant dans ce circuit de sortie, selon les relations :

$$I = k \cdot R$$

$$U = k \cdot R^2$$

k étant une constante pouvant être définie.

2. Appareil (1) selon la revendication 1, **caractérisé en ce que**, dans les canaux de sortie (9a, 9b, 9c), dans les chemins de courant (16a, 16b, 16c) conduisant aux électrodes de stimulation (6a, 6b, 6c), sont insérées des résistance de mesure (17a, 17b, 17c) pour la mesure du courant et, aux différentes résistances de mesure (17a, 17b, 17c) sont branchés respectivement des amplificateurs de mesure (18a, 18b, 18c), dont le signal de sortie est une mesure de l'intensité du courant de traitement et est introduit dans le circuit de micro-ordinateur (10), et à au moins un canal de sortie (9a, 9b, 9c) est prévu un circuit de mesure de tension (19a, 19b, 19c), qui envoie un signal de mesure au circuit de micro-ordinateur (10), qui correspond à la tension qui existe entre l'électrode de stimulation (6a, 6b, 6c) correspondant au canal de sortie concerné et un point de potentiel de référence (0) se trouvant dans le générateur de courant de traitement (3), qui est relié à une électrode de référence (7).

3. Appareil (1) selon la revendication 1 ou 2, **caractérisé en ce que**, dans le circuit de commande à courant adaptatif (22), est intégré un circuit de régulation à courant adaptatif (25), dans lequel est introduit, en tant que signal effectif, une valeur de mesure de résistance, pour la mesure de courant, provenant du signal de mesure du circuit de mesure de tension (19a, 19b, 19c) et du signal de sortie de l'amplificateur de mesure (18a, 18b), 18c).

4. Appareil (1) selon la revendication 1, **caractérisé en ce que** le circuit de commande (20, 21, 22) prévu dans le circuit de micro-ordinateur (10) introduit, dans les amplificateurs (15a, 15b, 15c) prévus dans les canaux de sortie, un signal de commande qui forme, selon une succession cyclique, des intervalles de temps (30), dans lesquels un flux de courant a lieu vers les électrodes de stimulation, et à des intervalles de temps (31), dans lesquels les canaux de sortie sont inactifs, un intervalle de flux de courant (30) et un intervalle de temps d'inactivité (31) formant ensemble un cycle de stimulation (32), et qui provoque une variation de la valeur moyenne de l'intensité, déterminée sur la durée d'un cycle de stimulation (32), en faisant varier cette durée par le signal de commande.

5. Appareil (1) selon la revendication 1, **caractérisé en ce que**, dans le circuit de micro-ordinateur (10), se trouve un circuit de commande à tension constante (20) activable sélectivement par un actionnement externe, qui est relié de manière fonctionnelle à une mémoire interne (11) pour les paramètres du courant de traitement, et qui introduit, par l'intermédiaire d'un convertisseur numérique-analogique (12), au moins dans un amplificateur (15a, 15b, 15c) se trouvant dans un canal de sortie (9a, 9b, 9c), un signal de commande qui influence, dans le circuit de sortie de cet amplificateur (15a, 15b, 15c), relié à une électrode de stimulation (6a, 6b, 6c), le comportement d'une source de tension constante dont la valeur de la tension est réglable.

6. Appareil (1) selon la revendication 2 et 5, **caractérisé en ce que**, dans le circuit de commande à tension constante (20), est intégré un circuit de régulation à tension constante (23) dans lequel est introduit le signal de mesure du circuit de mesure de tension (19a, 19b, 19c) en tant que signal effectif.

7. Appareil (1) selon la revendication 1 et 2, **caractérisé en ce que**, dans le circuit de micro-ordinateur (10), se trouve un circuit de commande à courant constant (21) activable sélectivement par un actionnement externe, qui est relié de manière fonctionnelle à une mémoire interne (11) pour les paramètres du courant de traitement et qui introduit, par l'intermédiaire d'un convertisseur numérique-analogique (12), au moins dans un amplificateur (15a, 15b, 15c) se trouvant dans un canal de sortie (9a, 9b, 9c), un signal de commande qui influence, dans le circuit de sortie de cet amplificateur (15a, 15b, 15c), relié à une électrode de stimulation (6a, 6b, 6c), le comportement d'une source de courant constant dont l'intensité est réglable, et, dans le circuit de commande à courant constant (21), est intégré un circuit de régulation à courant constant (24) dans lequel est introduit le signal de sortie de l'amplificateur de mesure (18a, 18b, 18c) en tant que signal effectif.

8. Appareil (1) selon l'une des revendications 1 à 7, **caractérisé en ce que** le circuit de commande (20, 21, 22) prévu dans le circuit de micro-ordinateur (10) introduit, dans les amplificateurs (15a, 15b, 15c) prévus dans les canaux de sortie, un signal de commande qui forme, selon une succession cyclique, des intervalles de temps (30), dans lesquels un flux de courant a lieu vers les électrodes de stimulation, et des intervalles de temps (31), dans lesquels les canaux de sortie sont inactifs, et, au cours de chaque intervalle de flux de courant (30), provoque un changement de polarité, un intervalle de flux de courant (30) et un intervalle d'inactivité (31) formant ensemble un cycle de stimulation (32) et, dans les intervalles de flux de courant (30), un courant de traitement s'écoule sous la forme d'un paquet d'impulsions successives (33) ou sous la forme d'impulsions individuelles (34).

9. Appareil (1) selon l'une des revendications précédentes, **caractérisé en ce que**, dans le circuit de micro-ordinateur (10) se trouve un circuit de commande (20, 21, 22) qui introduit, dans les amplificateurs (15a, 15b, 15c) se trouvant dans les canaux de sortie, un signal de commande qui forme, selon une succession cyclique, des intervalles de temps (30), dans lesquels un flux de courant a lieu vers les électrodes de stimulation, et des intervalles de temps (31), dans lesquels les canaux de sortie sont inactifs, un intervalle de flux de courant (30) et un intervalle d'inactivité (31) formant ensemble un cycle de stimulation (32), et qui, au cours des différents intervalles de flux de courant, provoque une ou plusieurs variations de l'intensité des courants de traitement introduits dans les différentes électrodes de stimulation, les variations d'intensité qui ont lieu au niveau de chacune des électrodes de stimulation (6a, 6b, 6c) considérées se produisant respectivement à contre-courant des variations d'intensité qui ont lieu au niveau d'une ou plusieurs autres électrodes de stimulation (n) considérées.

10. Appareil (1) selon la revendication 9, **caractérisé en ce que** trois canaux de sortie, correspondant chacun à une électrode de stimulation, sont prévus et, dans les intervalles de flux de courant, à chaque moment considéré, le courant de traitement introduit dans chacun électrode de stimulation (6a, 6b, 6c) est compensé en ce qui concerne l'intensité et la polarité par les courants de traitement introduits dans les deux autres électrodes de stimulation (6a, 6b, 6c).

11. Appareil (1) selon la revendication 9 ou 10, **caractérisé en ce que** les courants de traitement introduits dans les différentes électrodes de stimulation (6a, 6b, 6c) sont formés, dans chaque intervalle de flux de courant (30), par une impulsion individuelle (34) et, dans chacune de ces impulsions individuelles, sont prévues plusieurs variations d'intensité se succédant dans le temps et au moins un changement de polarité (37).

12. Appareil (1) selon l'une des revendications 1 à 11, **caractérisé en ce que** les variations uniques ou multiples de l'intensité du courant de traitement, prévues dans les intervalles de flux de courant (30), ont lieu par étapes.

13. Appareil (1) selon l'une des revendications 8 à 12, **caractérisé en ce que** le circuit de commande (20, 21, 22) prévu dans le circuit de micro-ordinateur (10) introduit, dans les amplificateurs (15a, 15b, 15c) se trouvant dans les canaux de sortie, un signal de commande qui forme, selon une succession cyclique, des intervalles de temps (30), dans lesquels un flux de courant a lieu vers les électrodes de stimulation, et des intervalles de temps (31), dans lesquels les canaux de sortie sont inactifs, un intervalle de flux de courant (30) et un intervalle d'inactivité (31) formant ensemble un cycle de stimulation (32), et au début (38) d'une série de cycles de stimulation constituée d'une pluralité de cycles de stimulation (32) successifs, pendant plusieurs cycles de stimulation successifs, l'intensité du courant de traitement introduit dans chacune des électrodes de stimulation (6a, 6b, 6c), considérée sous la forme d'une valeur moyenne calculée sur l'intervalle de flux de courant (30) correspondant, augmente de manière continue d'un cycle de stimulation (32) au cycle suivant puis maintient, pendant une pluralité de cycles de stimulation successifs, dans leurs intervalles de flux de courant, le niveau d'intensité des courants de traitement atteint auparavant.

14. Appareil (1) selon la revendication 13, **caractérisé en ce que**, à la fin (39) de la série de cycles de stimulation, le circuit de commande introduit, dans les amplificateurs se trouvant dans les canaux de sortie, un signal de commande qui diminue de manière continue l'intensité du courant de traitement introduit dans chacune des électrodes de stimulation (6a, 6b, 6c), considérée sous la forme d'une valeur moyenne calculée sur l'intervalle de flux de courant, d'un cycle de stimulation (32) au cycle de stimulation suivant.

15. Appareil (1) selon l'une des revendications 1 à 14, **caractérisé en ce que**, pour l'alimentation des amplificateurs (15a, 15b, 15c) se trouvant dans les canaux de sortie (9a, 9b, 9c), un convertisseur de tension (40) avec sortie bipolaire est prévu, qui est lui-même alimenté par la batterie (27) prévue dans l'appareil.

16. Appareil (1) selon la revendication 15, **caractérisé en ce que**, dans la liaison (41) d'alimentation conduisant de la batterie (27) au convertisseur de tension (40) est inséré un dispositif de commutation (42) qui coupe l'alimentation du convertisseur de tension (40) aussi bien pendant les pauses de flux de courant dans les circuits de courant de traitement que lors d'un dépassement d'une valeur limite prédéterminée du courant prélevé dans la batterie (27) par le convertisseur de tension (40).

**17.** Appareil (1) selon l'une des revendications 1 à 16, **caractérisé en ce que**, pour l'alimentation du circuit de micro-ordinateur (10), du convertisseur numérique-analogique (12) et des circuits de signaux de mesure (18a, 18b, 18c, 19a, 19b, 19c) prévus dans l'appareil (1), un convertisseur de tension (43) avec sortie unipolaire branché à la batterie (27) est prévu, qui est court-circuité par une diode Schottky (44), qui n'est activé que lors d'une baisse de la tension de la batterie et qui envoie, au niveau de sa sortie, une tension correspondant à la tension de consigne de la batterie.

# FIG. 1

FIG. 2

FIG. 3

FIG. 4

FIG. 5

FIG. 6

FIG. 8

FIG. 7

FIG. 10

EP 2 477 691 B1

# FIG. 9

# FIG. 11

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

* WO 8602567 A1

**In der Beschreibung aufgeführte Nicht-Patentliteratur**

* **VON SATOR-KATZENSCHLAGER S M et al.** The Short-and Long-Term Benefit in Chronic Low Back Pain Through Adjuvant Electrical Versus Manual Auricular Acupuncture. *Anesthesia and Analgesia,* vol. 98, 1359-1364